# EUROPÄISCHE PATENTSCHRIFT

(11) **EP 2 278 323 B1**
(45) Veröffentlichungstag und Bekanntmachung des Hinweises auf die Patenterteilung: **07.09.2011**
(21) Anmeldenummer: 10007225.5
(22) Anmeldetag: 13.07.2010
(51) Int. Cl.: G01N 27/74

(54) **Vorrichtung zum Bestimmen des Sauerstoffgehalts in einem Gas**
Device for determining the oxygen content of a gas
Dispositif de détermination de la teneur en oxygène dans un gaz

(30) Priorität: 16.07.2009 DE 102009033420
(43) Veröffentlichungstag der Anmeldung: 26.01.2011
(73) Patentinhaber: M & C TechGroup Germany GmbH, 40885 Ratingen (DE)
(72) Erfinder: Müller, Jörg, 21244 Buchholz (DE); Vonderschmidt, Stefan, 21073 Hamburg (DE)
(74) Vertreter: Berkenbrink, Kai-Oliver

(56) Entgegenhaltungen:
- EP-A1- 1 202 051
- DE-B3-102004 036 684
- DE-C1- 4 201 216
- STEFAN VONDERSCHMIDT ET AL: "A novel micro paramagnetic oxygen sensor" MICRO ELECTRO MECHANICAL SYSTEMS (MEMS), 2010 IEEE 23RD INTERNATIONAL CONFERENCE ON, IEEE, PISCATAWAY, NJ, USA, 24. Januar 2010 (2010-01-24), Seiten 903-906, XP031655073 ISBN: 978-1-4244-5761-8

## Beschreibung

Die Erfindung betrifft eine Vorrichtung zum Bestimmen des Sauerstoffgehalts in einem Gas mit einem Gaseinlass, einem Gasauslass und einem dazwischen angeordneten Messbereich.

Sensoren zur Bestimmung des Sauerstoffgehalts in Gasen können zum Beispiel auf dem Prinzip elektrochemischer Reaktionen, auf Festkörperionenleitung und dem Paramagnetismus beruhen.

Der letztgenannte Sensor beruht auf der paramagnetischen Eigenschaft des Sauerstoffs. Paramagnetische Stoffe werden in ein inhomogenes Magnetfeld hereingezogen, während diamagnetische Stoffe aus einem Magnetfeld verdrängt werden. Vorteil des paramagnetischen Verfahrens zur Sauerstoffmessung im Gegensatz zu anderen Verfahren ist, dass keine Stoffe verbraucht werden, keine chemischen Reaktionen stattfinden, und der Sensor damit langzeitstabil ist. Sauerstoffsensoren auf Basis der paramagnetischen Eigenschaften des Sauerstoffs sind bekannt und werden industriell hergestellt, und eingesetzt. Ein Beispiel hierfür ist die Sauerstoffmessung über ein Spiegelsystem. Eine frei schwingend angebrachte Hantel mit diamagnetischen Hantelkörpern wird durch ein sauerstoffhaltiges Gas aus ihrer Ruhelage ausgelenkt. Diese Auslenkung wird über einen Laser, einen Spiegel und eine Photodiode gemessen und ist ein Maß für die Sauerstoffkonzentration im Gas. Nachteile dieses Systems sind seine Größe sowie seine Anfälligkeit gegenüber Erschütterungen.

DE 42 01 216 C1 offenbart eine Vorrichtung zur Bestimmung des Sauerstoffgehaltes in einem Gas mit einem Gaseinlass, einem Gasauslass und einem dazwischen angeordneten Messbereich, wobei der Messbereich zwei nebeneinander angeordnete Kanäle aufweist.

Die Aufgabe der Erfindung besteht darin, einen auf dem Paramagnetismus beruhenden Sauerstoffsensor zu schaffen, der keinerlei bewegliche Teile und geringe Abmessungen hat und der sich durch eine höhere Empfindlichkeit auszeichnet.

Die erfindungsgemäße Lösung besteht, darin, dass der Messbereich drei nebeneinander angeordnete Kanäle in Form eines Hauptkanals, eines auf einer Seite des Hauptkanals angeordneten Messkanals und eines auf der anderen Seite des Hauptkanals angeordneten Referenzkanals, einen auf der vom Hauptkanal abgewandten Seite des Messkanals angeordneten Magneten mit einem inhomogenen Magnetfeld und Strömungssensoren im Messkanal und Referenzkanal aufweist.

Im Fall eines sauerstofffreien Gases bewegt sich der Hauptteil des Gasstroms zwischen Gaseinlass und Gasauslass durch den Hauptkanal, während durch den Messkanal und den Referenzkanal nur sehr wenig Gas strömt. Im Falle eines sauerstoffhaltigen Gases wird die Gasströmung im inhomogenen Magnetfeld des Magneten so abgelenkt, dass ein größerer Teil des Gases durch den Messkanal und ein kleinerer Teil durch den Referenzkanal strömt. Diese Strömungen im Messkanal und Referenzkanal werden durch die Strömungssensoren gemessen, und aufgrund dieser Messungen kann der Sauerstoffgehalt bestimmt werden.

Bei einer vorteilhaften Ausführungsform ist vorgesehen, dass auch im Hauptkanal ein Strömungssensor vorgesehen ist. Dabei sind insbesondere die Strömungssensoren als Widerstandthermometer ausgeführt. Diese beruhen darauf, dass bei unterschiedlichen Strömungsgeschwindigkeiten die Widerstände unterschiedlich stark abgekühlt werden, wodurch sich ihr Widerstand ändert. Der Vorteil dieser Widerstandsthermometer besteht dabei darin, dass sie sehr klein ausgebildet sein können.

Insbesondere ist die erfindungsgemäße Vorrichtung auch mit geringen Abmessungen, insbesondere auch mit den Methoden der Mikrosystemtechnik herstellbar, weist dabei aber doch eine industriell nutzbare hohe Empfindlichkeit auf. Vorteile eines mit Mikrosystemtechnik hergestellten paramagnetischen Sauerstoffsensors sind, neben den allgemeinen Vorteilen paramagnetischer Sensoren, die großen Miniaturisierungsmöglichkeiten sowie die durch einen Batchprozess bedingte hohe Reproduzierbarkeit und die geringen Herstellungskosten.

Bei einer vorteilhaften Ausführungsform ist die Vorrichtung als Glas-Silizium-Glas-Stapel ausgebildet. Auf einem Glassubstrat mit den Strukturen für die Widerstandsthermometer oder Thermoanemometer ist eine Schicht aus Silizium mit den Kanalstrukturen und eine weitere aus Glas als Deckel für die Kanalstrukturen angebracht.

Bei einer anderen vorteilhaften Ausführungsform besteht die Vorrichtung wenigstens teilweise aus Polymeren.

Als Widerstandsthermometer oder Thermoanemometer werden vorteilhafterweise Platin-Dünnschicht-Thermometer verwendet. Diese sind besonders empfindlich, wenn bei einer vorteilhaften Ausführungsform die Platin-Dünnschicht-Thermometer nasschemisch unterätzt sind, da so die Platin-Dünnschicht besonders empfindlich auf Änderungen der Strömungsgeschwindigkeit reagiert. Bei einer besonders vorteilhaften Ausführungsform sind die Widerstandsthermometer in der Mitte der Kanäle angeordnet, und zwar in Breitenrichtung und Höhenrichtung derselben, da hier die Strömungsgeschwindigkeit am größten ist.

Vorteilhafterweise sind die Widerstandsthermometer mit einer Messbrücke verbunden.

Bei einer kostengünstigen Ausführungsform ist der Magnet ein Permanentmagnet, der vorteilhafterweise aus Elementen der seltenen Erden besteht. Bei einer anderen Ausführungsform wird ein Elektromagnet verwendet. Dieser hat den Vorteil, dass es vorteilhafterweise vorgesehen werden kann, dass sein Erregerstrom moduliert wird. Die entsprechende Modulation der Strömungsgeschwindigkeiten erlaubt dann eine besonders genaue Messung des Sauerstoffgehalts. Anstelle der Modulation des Erregerstroms des Elektromagneten oder zusätzlich dazu kann vorgesehen sein, dass zwischen Gaseinlass und Messbereich eine Heizeinrichtung zur thermischen Modulation der paramagnetischen Eigenschaften des Gases angeordnet ist. Dabei wird die physikalische Tatsache ausgenutzt, dass der Paramagnetismus temperaturabhängig ist und die Suszeptibilität mit. steigender Temperatur abnimmt.

Bei einer Ausführungsform wird das Gas nicht nur vom Gaseinlass auf die drei Kanäle verteilt, sondern sind die drei Kanäle in Strömungsrichtung hinter den Widerstandsthermometern miteinander verbunden und münden in einem gemeinsamen Gasauslass. Bei einer anderen Ausführungsform lässt man das Gas frei am Ende der Kanäle ausströmen.

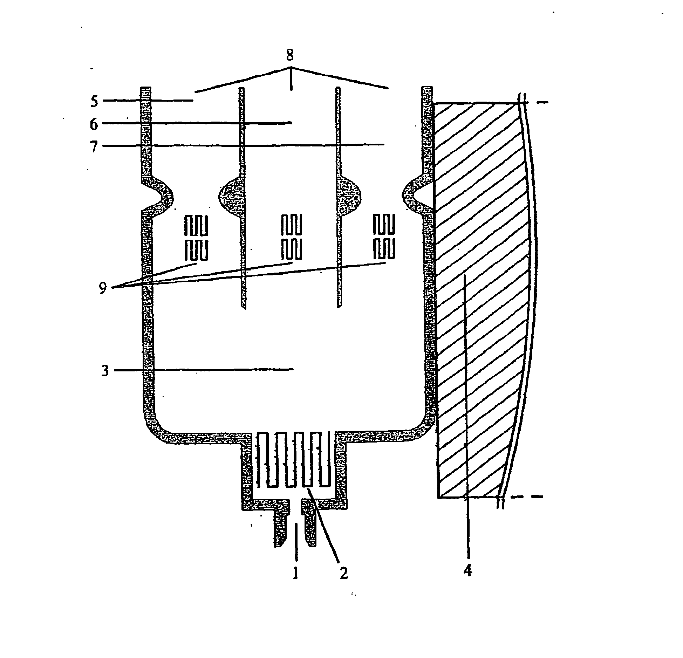

Die Erfindung wird im Folgenden anhand einer vorteilhaften Ausführungsform unter Bezugnahme auf die beigefügte Zeichnung beispielsweise beschrieben, die die Struktur einer erfindungsgemäßen Vorrichtung in Draufsicht zeigt.

Von einem Gaseinlass 1 wird das zu untersuchende Gas über eine Heizwendel 2 zur Modulation der paramagnetischen Eigenschaften des Gases in einen Bereich 3 geleitet, in dem es durch einen seitlich angeordneten Magneten 4, vorzugsweise aus den Elementen der seltenen Erden, abgelenkt werden kann. Durch die seitliche Anordnung ist die Größe des Magneten nicht durch die Größe des Mikrosystems begrenzt und kann somit beliebig groß bzw. stark gewählt werden. Alternativ zu einem Permanentmagneten besteht auch die Möglichkeit, das Magnetfeld mit einem Elektromagneten zu erzeugen und gegebenenfalls zu modulieren.

Anschließend an diesen Bereich strömt das Probengas durch drei Kanäle, einen Hauptkanal 6, einen Messkanal 7 und einen Referenzkanal 5 und kann anschließend den Sensor über einen Gasauslass 8 verlassen. Innerhalb des Messkanals 7 und des Referenzkanals 5 wird über Thermoanemometer 9 die Strömungsgeschwindigkeit des Gases in diesen Kanälen gemessen. Eine Messung der Strömungsgeschwindigkeit im Hauptkanal 6 über ein weiteres Thermoanemometer 9 ermöglicht die Bestimmung des Gesamtvolumenstroms.

Der Sensor lässt sich beispielsweise aus drei gestapelten Substraten herstellen, vorzugsweise einem aus Glas mit den Strukturen für die Thermoanemometer, einem aus Silizium mit den Kanalstrukturen und einem weiteren aus Glas als Deckel für die Kanalstrukturen. Aber auch andere Verfahren der Mikrosystemtechnik, insbesondere auch solche zur Abformung solcher (Teil-)Strukturen in Polymeren sind geeignet.

Für den Aufbau werden zuerst Platinleiterbahnen auf einem Glaswafer strukturiert. Um mit diesen Platinstrukturen die Strömungsgeschwindigkeit empfindlich messen zu können, werden sie z.B. zu freistehenden Platinfilamenten nasschemisch unterätzt.

In das Siliziumsubstrat werden vorzugsweise in einem ASE-Prozess (Advances Silicon Etching, fortgeschrittenes Siliziumätzen) die Kanalstrukturen geätzt und die beiden Substrate anodisch gebondet. Im weiteren Verlauf werden die Kanalstrukturen vollständig durch das Siliziumsubstrat geätzt. Abschließend wird dieser Verbund mit dem zweiten Glassubstrat verbunden, um verschlossene Kanalstrukturen zu erhalten.

Die erfindungsgemäße Vorrichtung arbeitet wie folgt. Das Probengas wird durch den Gaseinlass 1 in den Sensor geführt. Ist das Gas sauerstofffrei und somit diamagnetisch, durchströmt es das Feld 3 des Magneten 4 ungestört und fließt fast vollständig durch den breiten Hauptkanal 6. Den schmalen Messkanal 7 und den spiegelsymmetrisch dazu ausgelegten Referenzkanal 5 durchströmt nur eine sehr geringe Gasmenge. Die geringen Flüsse in diesen Kanälen begünstigen die erreichbare Empfindlichkeit bei der Messung sauerstoffhaltiger Gase. Sie sind der Kern der Erfindung. Durch den Gasauslass 7 tritt das Probengas aus dem Sensor aus. Ein sauerstoffhaltiges Gas wird im Magnetfeld 3 des Magneten 4 in Richtung zum Magneten 4 abgelenkt. Durch diese Richtungsänderung erhöht sich der Gasfluss im Messkanal 7, gleichzeitig sinkt er im Referenzkanal 5. Diese beiden Strömungsgeschwindigkeiten werden mit den Thermoanemometern 8 gemessen, sie korrelieren mit dem Sauerstoffgehalt des Gases. Für eine genaue Messung werden die Thermoelemente im Referenzkanal 5 und im Messkanal 7 vorzugsweise in einer Wheatstonschen Messbrücke verschaltet. Somit dient das Brückenausgangssignal als temperatur- und druckunabhängiges Messsignal.

Für eine weitere Erhöhung der Messempfindlichkeit kann die magnetische Suszeptibilität des Sauerstoffs über eine Heizung 2 moduliert werden. Eine geringe Wandstärke der Siliziumstrukturen dient zur thermischen Entkopplung des Sensors zur Umgebung.

### Bezugszeichenliste

- 1: Gaseinlass
- 2: Heizung
- 3: Einflussbereich des Magneten
- 4: Magnet
- 5: Referenzkanal
- 6: Hauptkanal
- 7: Messkanal
- 8: Gausaulass
- 9: Thermoanemometer

## Patentansprüche

1. Vorrichtung zum Bestimmen des Sauerstoffgehalts in einem Gas mit einem Gaseinlass (1), einem Gasauslass (8) und einem dazwischen angeordneten Messbereich, wobei der Messbereich drei nebeneinander angeordnete Kanäle in Form eines Hauptkanals (6), eines auf einer Seite des Hauptkanals (6) angeordneten Messkanals (7) und eines auf der anderen Seite des Hauptkanals (6) angeordneten Referenzkanals (5), einen auf der vom Hauptkanal (6) abgewandten Seite des Messkanals (7) angeordneten Magneten (4) mit einem inhomogenen Magnetfeld und Strömungssensoren (9) im Messkanal (7) und Referenzkanal (5) aufweist.

2. Vorrichtung nach Anspruch 1, **dadurch gekennzeichnet, dass** ein weiterer Strömungssensor (9) im Hauptkanal (6) angeordnet ist.

3. Vorrichtung nach Anspruch 1, **dadurch gekennzeichnet, dass** die Strömungssensoren (9) als Widerstandsthermometer ausgeführt sind.

4. Vorrichtung nach Anspruch 1, **dadurch gekennzeichnet, dass** der Messbereich (5, 6, 7) mit den Strömungssensoren (9) mit Mikrosystemtechnik hergestellt ist.

5. Vorrichtung nach Anspruch 1, **dadurch gekennzeichnet, dass** der Messbereich (5, 6, 7} als Glas-Silizium-Glas-Stapel ausgebildet ist.

6. Vorrichtung nach Anspruch 3, **dadurch gekennzeichnet, dass** die Widerstandsthermometer (9) Platin-Dünnschicht-Thermometer sind.

7. Vorrichtung nach Anspruch 6, **dadurch gekennzeichnet, dass** die Platin-Dünnschicht-Thermometer (9) unterätzt sind.

8. Vorrichtung nach Anspruch 1, **dadurch gekennzeichnet, dass** der Magnet (4) ein Permanentmagnet ist.

9. Vorrichtung nach Anspruch 8, **dadurch gekennzeichnet, dass** der Permanentmagnet (4) aus Elementen der Seltenen Erden besteht.

10. Vorrichtung nach Anspruch 1, **dadurch gekennzeichnet, dass** der Magnet (4) ein Elektromagnet ist.

11. Vorrichtung nach Anspruch 10, **dadurch gekennzeichnet, dass** sie eine Schaltung zur Modulation des Erregerstroms des Elektromagneten aufweist.

12. Vorrichtung nach Anspruch 1, **dadurch gekennzeichnet, dass** zwischen Gaseinlass (1) und Messbereich (5, 6, 7) eine Heizeinrichtung (2) zur thermischen Modulation der paramagnetischen Eigenschaften des Gases angeordnet ist.

## Claims

1. A device for determining the oxygen content in a gas with a gas inlet (1), a gas outlet (8) and a measurement region disposed in between, wherein the measurement region comprises three channels disposed beside one another in the form of a main channel (6), a measurement channel (7) disposed on one side of the main channel (6) and a reference channel (5) disposed on the other side of the main channel (6), a magnet (4) with an inhomogeneous magnetic field disposed on the side of the measurement channel (7) facing away from the main channel (6) and flow sensors (9) in the measurement channel (7) and reference channel (5).

2. The device according to claim 1, **characterised in that** a further flow sensor (9) is disposed in the main channel (6).

3. The device according to claim 1, **characterised in that** the flow sensors (9) are constituted as resistance thermometers.

4. The device according to claim 1, **characterised in that** the measurement region (5, 6, 7) with the flow sensors (9) is produced using microsystems technology.

5. The device according to claim 1, **characterised in that** the measurement region (5, 6, 7) is constituted as a glass-silicon-glass stack.

6. The device according to claim 3, **characterised in that** the resistance thermometers (9) are platinum thin-film thermometers.

7. The device according to claims 6, **characterised in that** the platinum thin-film thermometers (9) are under-etched.

8. The device according to claim 1, **characterised in that** the magnet (4) is a permanent magnet.

9. The device according to claim 8, **characterised in that** the permanent magnet (4) comprises elements of the rare earths.

10. The device according to claim 1, **characterised in that** the magnet (4) is an electromagnet.

11. The device according to claim 10, **characterised in that** it comprises a circuit for the modulation of the excitation current of the electromagnet.

12. The device according to claim 1, **characterised in that** a heating device (2) for the thermal modulation of the paramagnetic properties of the gas is disposed between the gas inlet (1) and the measurement region (5, 6, 7) .

## Revendications

1. Dispositif destiné à déterminer la teneur en oxygène dans un gaz comprenant une entrée de gaz (1), une sortie de gaz (8) et une zone de mesure intercalée, la zone de mesure présentant trois canaux disposés les uns à côté des autres sous la forme d'un canal principal (6), d'un canal de mesure (7) disposé sur un côté du canal principal (6) et d'un canal de référence (5) disposé sur l'autre côté du canal principal (6), un aimant (4) disposé sur le côté, opposé au canal principal (6), du canal de mesure (7) avec un champ magnétique non homogène et des capteurs d'écoulement (9) dans le canal de mesure (7) et le canal de référence (5).

2. Dispositif selon la revendication 1, **caractérisé en ce qu'**un autre capteur d'écoulement (9) est disposé dans le canal principal (6).

3. Dispositif selon la revendication 1, **caractérisé en ce que** les capteurs d'écoulement (9) sont conçus sous forme de thermomètres à résistance.

4. Dispositif selon la revendication 1, **caractérisé en ce que** la zone de mesure (5, 6, 7) avec les capteurs d'écoulement (9) est fabriquée dotés de technique de microsystème.

5. Dispositif selon la revendication 1, **caractérisé en ce que** la zone de mesure (5, 6, 7) est conçue sous forme de pile verre-silicium-verre.

6. Dispositif selon la revendication 3, **caractérisé en ce que** les thermomètres à résistance (9) sont des thermomètres à couche mince de platine.

7. Dispositif selon la revendication 6, **caractérisé en ce que** les thermomètres à couche mince de platine (9) sont soumis à une attaque sous-jacente.

8. Dispositif selon la revendication 1, **caractérisé en ce que** l'aimant (4) est un aimant permanent.

9. Dispositif selon la revendication 8, **caractérisé en ce que** l'aimant permanent (4) est constitué d'éléments des terres rares.

10. Dispositif selon la revendication 1, **caractérisé en ce que** l'aimant (4) est un électroaimant.

11. Dispositif selon la revendication 10, **caractérisé en ce qu'**il présente un circuit pour la modulation du courant d'excitation de l'électroaimant.

12. Dispositif selon la revendication 1, **caractérisé en ce qu'**un dispositif de chauffage (2) pour la modulation thermique des propriétés paramagnétiques du gaz est disposé entre l'entrée de gaz (1) et la zone de mesure (5, 6, 7) .
